# EUROPÄISCHE PATENTSCHRIFT

(11) **EP 0 532 867 B1**
(45) Veröffentlichungstag und Bekanntmachung des Hinweises auf die Patenterteilung: **05.03.1997**
(21) Anmeldenummer: 92112903.7
(22) Anmeldetag: 29.07.1992
(51) Int. Cl.: C12P 13/04, C12P 13/08

(54) **Verfahren zur fermentativen Herstellung von Aminosäuren**
Process for the fermentative production of amino acids
Procédé de production d'amino-acides par fermentation

(30) Priorität: 17.09.1991 DE 4130867
(43) Veröffentlichungstag der Anmeldung: 24.03.1993
(73) Patentinhaber: DEGUSSA AG, 60311 Frankfurt (DE)
(72) Erfinder: Pfefferle, Walter, Dr., W-4802 Halle (DE); Lotter, Hermann, Dr., W-6452 Hainburg (DE); Friedrich, Heinz, Dr., W-6450 Hanau 9 (DE); Degener, Wolfgang, Dr., W-4250 Melle 1, Ortsteil Eicken-Bruche (DE)

(56) Entgegenhaltungen:
- DD-A- 268 834
- DD-A- 269 167
- FR-A- 2 488 273
- FR-A- 2 604 447

## Beschreibung

Die Erfindung betrifft ein verbessertes Verfahren zur fermentativen Herstellung von Aminosäuren wie z. B. L-Lysin oder L-Threonin.
L-Lysin ist eine essentielle Aminosäure und wird in großem Umfang als Tierfuttersupplement eingesetzt.

Die Gewinnung einer Anzahl von Aminosäuren erfolgt im allgemeinen auf dem biosynthetischen Weg und ist aus dem Stand der Technik seit langem bekannt. Die als Produzenten verwendeten Bakterienstämme zeichnen sich durch die Fähigkeit aus, diese Aminosäuren in kurzer Zeit in hohen Konzentrationen in das Kulturmedium auszuscheiden. Zur Vermeidung hoher Anfangskonzentration an Substrat werden in der Regel Fed-Batch Prozesse durchgeführt.
Durch die sehr hohe Stoffwechselkapazität der verwendeten Produktionsstämme ist es von entscheidender Bedeutung, den Fermentationsprozeß so zu führen, daß die Maximalwerte an Sauerstoffbedarf und Wärmeentwicklung eine wirtschaftlich vertretbare Größenordnung aufweisen.
Daher werden verschiedene Strategien angewandt, die die metabolische Aktivität der Organismen so regeln, daß einerseits die Sauerstoffversorgung und Wärmeabfuhr gewährleistet ist, andererseits die Verteilung von Biomasse- und Produktbildung ausbalanciert ist.

Aus CSFR-PS 212 558 ist ein Verfahren mit diskontinuierlicher Zufütterung bekannt. bei dem die metabolische Aktivität in der Wachstumsphase über ph-Änderungen, der Gesamtgehalt an Biomasse über α-Aminostickstoff eingestellt wird.
In der SU-PS 1 157 059 wird ein Verfahren mit diskontinuierlicher Zufütterung beschrieben, bei dem die Threoninkonzentration als Kriterium für die Zufütterung dient, und der Gehalt der reduzierenden Verbindung auf 3 bis 5 % gehalten wird.
Ein sehr fein abgestimmtes Verfahren ist aus der EP-B-0 122 163 bekannt. Bei diesem Prozeß liegen 2. kontinuierlich zuzugebende Feed-Lösungen vor: Eine Leucinphosphatlösung, die so zudosiert wird, daß über die Supplementzugabe, sowohl die Intensität des Stoffwechsels als auch die Bildung der Biomasse limitiert werden. Die zweite Feed-Lösung, eine Zuckerlösung, wird so zugefüttert, daß die aktuelle Zuckerkonzentration zwischen 5 und 15 g/l gehalten wird. Daraus wird ersichtlich, daß die Kultur aufgrund einer Limitierung durch die Supplemente Leucin/Phosphat während der Zufütterungsphase zu jedem Zeitpunkt weniger Zucker verbraucht, als im Kulturmedium zur Verfügung steht.
Diese Verfahrensweise ist kongruent mit der mehrfach dokumentierten Ansicht, daß sowohl eine C-Limitierung als auch ein starker C-Überschuß vermieden werden sollte.
(z. B. DD-PS 269 167); Hadj Sassi et al., "Biotechn. Letters, Vol. 10, No. 8, Seite 583 - 586 (1988) schlagen hierfür sogar 90 bis 140 g/l Glucose vor. Die metabolische Aktivität wird folglich immer durch eine andere Größe als die der C-Quelle reguliert.

Aufgabe der Erfindung ist ein Verfahren zur fermentativen Herstellung von Aminosäuren, das unter einem höheren Umsetzungsgrad der eingesetzten Kohlenstoffquelle (Zucker) verläuft, und bei dem man in der biomassefreien Trockenmasse eine höhere Aminosäurenkonzentration erzielt.

Gegenstand der Erfindung ist ein Verfahren zur fermentativen Herstellung von Aminosäuren, bei dem man einen eine oder mehrere Aminosäuren produzierenden Stamm der Gattungen Brevibacterium oder Corynebacterium in einem Nährmedium züchtet und am Ende der Fermentation die Aminosäure(n) aus der Kulturflüssigkeit isoliert, dadurch gekennzeichnet, daß der Bakterienkultur im Anschluß an die logarithmische Wachstumsphase (in der Produktionsphase) weniger an verwertbarer Kohlenstoffquelle, zur Verfügung steht als sie infolge derStammkonstruktion und der im Nährmedium bereitgestellten Menge der ansonsten notwendigen Supplemente metabolisieren könnte.
Das Fermentations-(Nähr-)medium ist ansonsten wie allgemein bekannt zusammengesetzt.
Es enthält neben Kohlenstoffquellen wie assimilierbaren Zuckern, Saccharose, Glucose, Melassen oder Stärkehydrolysaten und Ammoniumionen bei auxotrophen Produzenten komplexe Bestandteile als Quelle für die aufgrund einer oder mehrerer Auxotrophien notwendigen organischen Supplemente, beispielsweise Proteinhydrolysate als α-Aminostickstoffquelle, Vitamine sowie anorganische Salze. Bei dem starken Wachstum zu Beginn der Fermentation handelt es sich im allgemeinen um eine logarithmische Wachstumsphase. Diese kann nach Bedarf durch Limitierung der Supplemente und/oder der Kohlenstoffquelle abgekürzt werden.

Auch im Anschluß daran gibt es noch ein Zellenwachstum. Dessen Umfang beläuft sich jedoch nur auf einen geringen Bruchteil der als logarithmisch gekennzeichneten Phase.
Bevorzugt eingesetzt werden Stämme, die L-Lysin und/oder L-Threonin produzieren.
Das Fermentationsmilieu wird so gewählt, daß die Temperatur zwischen 25 und 40 °C, vorzugsweise 30 bis 36 °C, der pH-Wert zwischen 6 und 8, vorzugsweise 7 bis 7,5 und die Ammoniumionenkonzentration zwischen 0,5 und 8 g/l liegen. Die Brühe wird gerührt und ausreichend mit Sauerstoff versorgt.
Insbesondere bei Aminosäure-auxotrophen Lysinausscheidern läßt sich die Metabolisierung des Zuckers über die Menge der zuzugebenden Aminosäure steuern.
Deren Konzentration bzw. die Konzentration anderer notwendiger Supplemente beläuft sich nach der Wachstumsphase vorteilhaft auf je 0 bis 0,1 g/l, insbesondere 0 bis 0,05 g/l.
So wird beispielsweise bei einem Leucin-auxotrophen Lysinausscheider das Zucker/Leucin-Verhältnis in einem kontinuerlich zuzugebenden Zufütterungsmedium so gewählt, daß man die Biomassebildung über die Leucinversorgung limitiert, gleichzeitig jedoch nur einen Bruchteil des Zuckers anbietet, der mit der gegebenen Leucinkonzentration umgesetzt werden könnte.

Vorteilhaft beläuft sich die Konzentration an verwertbaren Zuckern im Anschluß an die starke Wachstumsphase auf 0 bis <3 g/l, insbesondere jedoch auf 0 bis 1 g/l.

Die Konzentration 0 g/l in der Fermentationsbrühe bedeutet jedoch sowohl in Bezug auf eventuell notwendige Supplemente als auch die Kohlenstoffquelle nicht, daß diese Substanzen nicht kontinuierlich zugeführt werden.
Es heißt vielmehr, daß man diese Verbindungen in einer Menge zuführt, die unmittelbar von den Bakterienkulturen aufgenommen wird.
Diese nach dem erfindungsgemäßen Verfahren durchgeführte Fermentation weist eine Reihe von ganz entscheidenden Vorteilen gegenüber den oben aufgeführten, herkömmlichen Prozessen auf:
1. Die metabolische Aktivität und somit der Sauerstoffbedarf und die Wärmeentwicklung der Kultur können direkt und ohne Verzögerung über die Dosierrate der Zufütterung beeinflußt und an die Fermenterkapazität angepaßt werden.
2. Die Fermentationsbrühen zeichnen sich durch einen höheren Produktgehalt in der Gesamttrockenmasse und damit durch eine größere Reinheit aus.
   Dadurch, daß der Bakterienkultur über den gesamten Zufütterungszeitraum weniger Substrat angeboten wird als sie umsetzen könnte, die Kohlenstoffquelle somit die primäre Limitierung darstellt, wird ein Abfließen in Richtung Nebenprodukte verhindert.
3. Die Fermentationen weisen gegenüber Fermentationsläufen mit primärer Limitierung über Supplemente eine höhere Ausbeute auf.
4. Bei einem Produkt-Monitoring kann die Fermentation direkt und ohne Nachlaufzeit im Optimum bzw. auf einem Plateau abgebrochen werden, die Brutto-Ausbeute entspricht zu jedem Zeitpunkt der Netto-Ausbeute.
5. Bei einem Aufarbeitungskonzept, das eine Direkteindampfung der Fermentationsbrühe vorsieht, kann der Fermenterinhalt bei technischen Störungen sofort der Aufarbeitung zugeführt werden, ohne daß durch einen hohen Restzuckergehalt die Produktqualität beeinträchtigt wird.

Die nachfolgenden Beispiele dokumentieren Ausführungsmöglichkeiten des erfindungsgemäßen Verfahrens.

### Beispiele

### Beispiel 1 (Vergleichsbeispiel)

In einem Fermentationsbehälter mit Rührwerk und Belüftungssystem wurden 5,1 kg einer sterilen Lösung vorgelegt, die folgende Komponenten enthält

| | |
|---|---|
| Wasser | 4540 g |
| Melasse | 26 g |
| Glucose | 125 g |
| Maiskleberhydrolysat (schwefelsauer) | 35 g |
| Hydrolysat der Produzentenbiomasse (schwefelsauer) | 320 g |
| Ammoniumsulfat | 45 g |
| Phosphorsäure 85 % | 7 g |
| Magnesiumsulfat | 3 g |

weitere Mineralsalze, Spurenstoffe sowie Biotin und Thiamin
und mit Ammoniaklösung auf einen pH-Wert von 7,3 eingestellt wurde. Zu dieser Lösung wurden bei 33 bis 35 °C 0,6 1 einer Impfkultur eines Corynebacterium glutamicum DM 346-1 gegeben, das die genetischen Marker leu⁻, oxalysinresistent und aminoaethylresistent trägt. Die Impfkultur wurde bereitet durch 15-stündige Inkubation bei 33 °C und pH 7 unter Rühren und Belüftung in einem Medium mit 4,4 Massenprozent Melassezusätzlich 2 % Saccharose, und 14 % Sojamehlhydrolysat (schwefelsauer) unter Zugabe von 3 % Ammoniumsulfat, 0,05 % Phosphorsäure und 0,02 % Magnesiumsulfat sowie den Vitaminen Biotin und Thiamin.

Bei ausreichender Rührung, Belüftung und einer pH-Justierung auf einen Wert von ca. 7,3 mittels wässriger Ammoniaklösung wurde nach dem Ende der logarithmischen Wachstumsphase im Hauptfermenter folgendes mit wässriger Ammoniaklösung neutralisierte Medium innerhalb von 32 h nach herkömmlicher Art kontinuierlich so zudosiert, daß die in der Fermentationsbrühe meßbare Zuckerkonzentration zwischen 5 und 35 g/l (enzymatische Bestimmungen auf Saccharose und Glucose) zu liegen kam:

| | |
|---|---|
| Wasser | 1250 g |
| Melasse | 94 g |
| Glucose | 1465 g |
| Maiskleberhydrolysat (schwefelsauer) | 39 g |
| Hydrolysat der Produzentenbiomasse (schwefelsauer) | 265 g |
| Ammoniumsulfat | 31 g |
| Phosphorsäure 85 % | 4 g |
| Magnesiumsulfat | 2 g |

weitere Mineralsalze, Spurenstoffe sowie Biotin und Thiamin.
Am Endpunkt der Fermentation, nachdem der gesamte assimilierbare Zucker im Fermentationsmedium aufgebraucht war, lag der Konversionsgrad Zucker in Lysin bei 35 %,berechnet als LysxHCl, der Lysinbasegehalt der biomassefreien eingedampften Fermentationslösung bei 45 %.

### Beispiel 2

Die Inokulumsbereitung, das im Hauptfermenter vorgelegte Medium, sowie die Kulturbedingungen entsprechen dem in Beispiel 1 dargestellten Bedingungen.
Auch das Medium 2 besteht bis auf folgende Modifikation aus denselben Bestandteilen:

| | |
|---|---|
| Wasser | 1560 g |
| Melasse | 75 g |
| Glucose | 1170 g |

Bei diesem Versuch wurde das Zufütterungsmedium mit derselben Dosierrate wie in Beispiel 1 zugegeben. Die Verlaufsanalysen auf assimilierbaren Zucker zeigten, entsprechend dem hier beanspruchten erfindungsgemäßen Verfahren, daß während der gesamten Zufütterungsdauer die meßbare Konzentration an assimilierbaren Zuckern unter 3 g/l, jedoch fast ausschließlich unter 1 g/l gehalten wurde. Die Analysen auf Leucin in der Fermentationsbrühe mittels Aminosäureanalysator zeigten, daß während der Zufütterung nach Aufbrauchen der im Medium 1 vorgelegten Leucinmenge zu keinem Zeitpunkt eine Leucinkonzentration größer als 0,05 g/l gemessen wurde.

Nach Fermentationsende betrug der Koversionsgrad Zucker in Lysin (berechnet als Lys∗HCl) 40 %, der Lysinbasegehalt der biomassefreien eingedampften Fermentationsbrühe lag bei 54 %.

### Beispiel 3

In einem 10 m³-Reaktor werden 3980 kg eines sterilen Medium vorgelegt, das folgende Zusammensetzung aufweist:

| | |
|---|---|
| Saccharose | 320 kg |
| Melasse | 20 kg |
| Maiskleberhydrolysat | 230 kg |
| 25 % wässriges Ammoniumsulfat | 150 kg |
| Citronensäure * H₂O | 2,3 kg |
| Phosphorsäure (89 %) | 6,6 kg |
| MgSo₄*7H₂O | 2,8 kg |
| CaCl₂*2H₂O | 75 g |
| FeSO₄*H₂O | 113 g |
| MnSO₄*H₂O | 113 g |
| ZnSO₄*7H₂O | 5,6 g |
| CuSO₄*5H₂O | 0,6 g |
| Biotin | 1,1 g |
| Thiamin*HCl | 0,8 g |
| NH₄OH (2-3 %) | 1010 kg |
| Wasser | 2258 kg |
| pH: 7,0 | |

Der Behälter wird bei 33 °C gerührt und ausreichend belüftet. Nach Transfer von 250 l Inokulum des Stammes DM 282-2, der die genetischen Marker leucinauxotroph und aminoaethylcysteinresistent trägt (nach 16 h Inkubation in einem Medium mit 6 % Melasse, 14 % Sojamehlhydrolysat, 1 % Ammmoniumsulfat und 0,1 % Phosphorsäure bei pH 7 und 30 °C) in den 10 m³-Reaktor wird der pH durch wässrigen Ammoniak bei 7,0 gehalten. die Belüftung wird so eingestellt, daß der Gelöstsauerstoff immer über 15 % Sättigung zu liegen kömmt.

Nachdem die Kultur auf eine optische Dichte (535 nm) von ca. 30 angewachsen ist, wird ein Produktionsmedium mit folgender Zusammensetzung mit einer Dosierrate von 30 l/h zudosiert.

| | |
|---|---|
| Saccharose | 940 kg |
| Melasse | 50 kg |
| Maiskleberhydrolysat | 180 kg |
| 25 % wässriges Ammoniumsulfat | 80 kg |
| Citronensäure * H₂O | 1 kg |
| Phosphorsäure (89 %) | 2,8 kg |
| MgSo₄*7H₂O | 1,2 kg |
| FeSO₄*H₂O | 48 g |
| MnSO₄*H₂O | 48 g |
| ZnSO₄*7H₂O | 2,4 g |
| CuSO₄*5H₂O | 0,3 g |
| Biotin | 0,6 g |
| Thiamin*HCl | 0,4 g |
| NH₄OH (25 %) | 80 kg |
| Wasser | 740 kg |
| pH: 7,5 | |

Während der Produktionsphase wird der pH-Wert auf 7,3 gehalten. Nach dem Aufbrauchen des im Wachstumsmedium vorgelegten Zuckers wurde gemäß der hier beanspruchten Erfindung während der Zufütterungsphase eine Konzentration an assimilierbarem Zucker von 1 g/l nicht überschritten, die meßbare Leucinkonzentration lag unter 0,05 g/l.
Bei Fermentationsende lag der Konversionsgrad Zucker in Lysin (als Lys∗HCl) bei 32,3 %, der Lysin-Base-Gehalt der biomassefreien eingedampften Fermentationsbrühe bei 54,7 %.

### Beispiel 4 (Vergleichsbeispiel)

Die Inokulumsherstellung, die Verfahrensparameter, sowie die Medien in der Wachstumsphase und in der Produktionsphase entsprechen den unter Beispiel 3 aufgeführten Bedingungen. Allerdings wurde numehr mit einer Dosierrate von ca. 100 l/h zugefüttert. Dadurch lagen nach Aufbrauchen der im Wachstumsmedium vorgelegten Zuckermenge die meßbaren Konzentrationen an assimilierbarem Zucker während des Zufütterungszeitraumes immer deutlich über 5 g/l, die meßbare Konzentration an Leucin blieb jedoch nach wie vor unter 0,05g/l. Der Konversionsgrad Zucker in Lysin (berechnet als Lys∗HCl) lag am Fermentationsende bei 30,9 %, der Lysinbasegehalt der biomassefreien Fermentationsbrühe bei 43,5 %.

### Beispiel 5 (Vergleichsbeispiel)

Das Anzucht-, das Wachstums- und das Produktionsmedium entsprechen in ihrer Zusammensetzung den Medien des Beispiels 1, mit dem Unterschied, daß die Glucose im Wachstumsmedium durch 25 g/l Saccharose, im Produktionsmedium durch 564 g/l Saccharose ersetzt wurde; die Inkubationsparameter incl. der Inokulumsbereitung sind ebenfalls identisch.
In einem Kleinfermenter mit Rührwerk und Belüftung wurden 0,82 kg (0,8 l) des sterilen Wachstumsmediums vorgelegt.
Zu dieser Lösung wurden bei 33 bis 35 °C 0,1 l einer Impfkultur des Corynebacterium glutamicum DSM 5715 gegeben. Nach Erreichen einer optischen Dichte von ca. 30 (535 nm) wurden 533 g (430 ml) des Produktionsmediums innerhalb von 24 h kontinuierlich zudosiert.

Während der Zufütterung lag der meßbare Zuckergehalt immer über 5 g/l im Fermentationsmedium, der Gehalt an Leucin nach Aufbrauchen der im Wachstumsmedium vorgelegten Menge immer unter 0,05 g/l.
Nach Fermentationsende wurden im Medium 74 g Lysin als LysxHCl detektiert, was bei einem Gesamtsaccharoseeinsatz von 275 g einem Konversionsgrad von 27 % entspricht. Der Lysingehalt der Gesamtrockenmasse lag bei 30,5 %.

### Beispiel 6

In einem Versuch ebenfalls mit dem Stamm DSM 5715, in dem alle Medien- und Inkubationsparameter mit den Bedingungen von Versuch 5 identisch waren, wurde das Produktionsmedium nunmehr innerhalb von 39 h kontinuierlich zudosiert.
Gemäß des hier beanspruchten Verfahrens lag im Zufütterungszeitraum nach Aufbrauchen der im Wachstumsmedium vorgelegten C- und Leucinquelle die aktuelle Saccharosekonzentration unter 1 g/l, die Leucinkonzentration unter 0,05 g/l. Bei Fermentationsende wurden im Medium 89 g Lysin (als LysinxHCl) detektiert, der Konversionsgrad lag bei 32 %.
Der Lysinbasegehalt in der Gesamttrockenmasse betrug 36,3 %.

## Patentansprüche

1. Verfahren zur fermentativen Herstellung von Aminosäuren, bei dem man einen eine oder mehrere Aminosäuren produzierenden Stamm der Gattungen Brevibacterium oder Corynebacterium in einem Nährmedium züchtet und am Ende der Fermentation die Aminosäure(n) aus der Kulturflüssigkeit isoliert,
dadurch gekennzeichnet, daß der Bakterienkultur im Anschluß an die logarithmische Wachstumsphase weniger an assimilierbarer Kohlenstoffquelle zur Verfügung steht als sie infolge der Stammkonstruktion und der im Nährmedium bereitgestellten Menge der ansonsten notwendigen Supplemente metabolisieren könnten, und sich die Konzentration der Kohlenstoffquelle auf 0 bis <3 g/l beläuft.

2. Verfahren gemäß Anspruch 1,
dadurch gekennzeichnet, daß man auxotrophe Bakterienstämme einsetzt, und mindestens eine der aufgrund einer oder mehrerer Auxotrophien zuzudosierenden organischen Verbindungen nach der logarithmischen Wachstumsphase auf eine Konzentration von je 0 bis 0,1 g/l begrenzt.

3. Verfahren gemäß einem oder mehreren der Ansprüche 1 oder 2,
dadurch gekennzeichnet, daß man L-Lysin und/oder L-Threonin produzierende Stämme einsetzt.

4. Verfahren gemäß Anspruch 3,
dadurch gekennzeichnet, daß man Leucin-auxotrophe Stämme von Corynebacterium glutamicum einsetzt.

5. Verfahren gemäß Anspruch 4,
dadurch gekennzeichnet, daß man die Biomassebildung über die Leucinversorgung limitiert.

## Claims

1. A process for fermentative preparation of amino acids, in which a strain of the genera Brevibacterium or Corynebacterium producing one or more amino acids is cultivated in a nutrient medium and the amino acid(s) is/are isolated from the culture fluid at the end of fermentation, characterised in that after the logarithmic growth phase, the bacterial culture has a smaller quantity of assimilable source of carbon available to it than it could metabolize on the basis of the structure of the strain and the quantity of other necessary supplements provided in the nutrient medium, and the concentration of the carbon source varies from 0 to <3g/l.

2. A process according to Claim 1, characterized in that auxotrophic bacterial strains are used and at least one of the organic compounds which are to be added due to the single or multiple auxotrophy is limited to a concentration of from 0 to 0.1 g/l after the logarithmic growth phase.

3. A process according to Claim 1 or 2, characterised in that strains producing L-lysine and/or L-threonine are used.

4. A process according to Claim 3, characterised in that leucine-auxotrophic strains of Corynebacterium glutamicum are used.

5. A process according to Claim 4, characterised in that the formation of biomass is limited via the supply of leucine.

## Revendications

1. Procédé de préparation par fermentation d'acides aminés, dans lequel on cultive une souche produisant un ou plusieurs acides aminés des types Brevibacterium ou Corynebacterium dans un milieu nutritif et, à la fin de la fermentation, on isole le ou les acides aminés du liquide de culture, caractérisé en ce que la culture bactérienne dispose, à la suite de la phase de croissance logarithmique, de moins de source de carbone assimilable qu'elle ne pourrait en métaboliser du fait de la structure de la souche et de la quantité, mise à disposition dans le milieu nutritif, des suppléments autrement nécessaires, et que la concentration de la source de carbone est de 0 à <3 g/l.

2. Procédé selon la revendication 1, caractérisé en ce qu'on utilise des souches bactériennes auxotrophes et on restreint au moins un des composés organiques à ajouter par dosage sur base d'un ou plusieurs auxotrophes, après la phase de croissance logarithmique, à une concentration de 0 à 0,1 g/l.

3. Procédé selon une ou plusieurs des revendications 1 et 2, caractérisé en ce qu'on utilise des souches produisant de la L-lysine et/ou de la L-thréonine.

4. Procédé selon la revendication 3, caractérisé en ce qu'on utilise des souches auxotrophes de leucine de Corynbacterium glutamicum.

5. Procédé selon la revendication 4, caractérisé en ce qu'on limite la formation de biomasse par alimentation de leucine.
